(19) **Europäisches Patentamt**

**European Patent Office**

**Office européen des brevets**

(11) **EP 1 006 863 B1**

(12) **EUROPEAN PATENT SPECIFICATION**

(45) Date of publication and mention
of the grant of the patent:
**15.10.2003 Bulletin 2003/42**

(51) Int Cl.⁷: **A61B 5/00**

(86) International application number:
**PCT/IB97/00287**

(21) Application number: **97906319.5**

(22) Date of filing: **21.03.1997**

(87) International publication number:
**WO 98/042249 (01.10.1998 Gazette 1998/39)**

(54) **METHOD AND APPARATUS FOR ADAPTIVELY AVERAGING DATA SIGNALS**

VERFAHREN UND GERÄT ZUR ADAPTIVEN MITTELWERTBILDUNG VON DATENSIGNALEN

PROCEDE ET APPAREIL D'ETABLISSEMENT ADAPTATIF DE MOYENNE DE SIGNAUX DE DONNEES

(84) Designated Contracting States:
**AT BE CH DE DK ES FI FR GB GR IE IT LI LU MC NL PT SE**

(43) Date of publication of application:
**14.06.2000 Bulletin 2000/24**

(73) Proprietor: **Nellcor Puritan Bennett Incorporated Pleasanton, CA 94588 (US)**

(72) Inventors:
• **BAKER, Clark, R., Jr.**
  **Castro Valley, CA 94546 (US)**
• **YORKEY, Thomas, J.**
  **San Ramon, CA 94583 (US)**

(74) Representative: **Belcher, Simon James**
  **Urquhart-Dykes & Lord**
  **Tower House**
  **Merrion Way**
  **Leeds LS2 8PA (GB)**

(56) References cited:
**EP-A- 0 335 357**      **EP-A- 0 586 025**
**WO-A-91/11137**      **US-A- 4 407 290**
**US-A- 4 960 126**      **US-A- 5 355 882**

## Description

This invention relates to a method and apparatus for measuring physiological parameters, in particular for reducing noise effects in a system for measuring a physiological parameter. It relates in particular to a method and apparatus for adaptively averaging data signals. The invention employs filtering techniques in pulse oximetry to estimate the oxygen saturation of haemoglobin in arterial blood.

Pulse oximeters typically measure and display various blood flow characteristics including but not limited to the oxygen saturation of haemoglobin in arterial blood. Oximeters pass light through blood perfused tissue such as a finger or an ear, and optically sense the absorption of light in the tissue. The amount of light absorbed is then used to calculate the amount of the blood constituent (e.g., oxyhaemglobin) being measured.

The light passed through the tissue is selected to be of one or more wavelengths that are absorbed by the blood in an amount representative of the amount of the blood constituent present in the blood. The amount of light passed through the tissue varies in accordance with the changing amount of blood constituent in the tissue and the related light absorption. The calculation of saturation can then be based on Beer-Lambert's law. Traditionally, the determination of saturation measures light absorption at two wavelengths, for example red and infra red. Saturation can then calculated by solving for the "ratio of ratios", as disclosed in US-4802486, US-4911167, US-4928692, US-4934372, US-4869254, US-5078136 and US-5485847.

The optical signal through the tissue can be degraded by both noise and motion artifact. One source of noise is ambient light which reaches the light detector. Another source of noise is electromagnetic coupling from other electronic instruments. Motion of the patient also introduces noise and affects the signal. For example, the contact between the detector and the skin, or the emitter and the skin, can be temporarily disrupted when motion causes either to move away from the skin. In addition, since blood is a fluid, it responds differently than the surrounding tissue to inertial effects, thus resulting in momentary changes in volume at the point to which the oximeter probe is attached.

Motion artifact can degrade a pulse oximetry signal relied upon by a physician, without the physician's awareness. This is especially true if the monitoring of the patient is remote, the motion is too small to be observed, or the doctor is watching the instrument or other parts of the patient, and not the sensor site.

An oximeter system is disclosed in US-5025791 in which an accelerometer is used to detect motion. When motion is detected, readings influenced by motion are either eliminated or indicated as being corrupted. In a typical oximeter, measurements taken at the peaks and valleys of the blood pulse signal are used to calculate the desired characteristic. Motion can cause a false peak, resulting in a measurement having an inaccurate value and one which is recorded at the wrong time.

Another system is disclosed in US-4802486 in which an EKG signal is monitored and correlated to the oximeter reading to provide synchronization to limit the effect of noise and motion artifact pulses on the oximeter readings. This reduces the chances of the oximeter locking onto a periodic motion signal.

The system disclosed in US-5078136 involves the use of linear interpolation and rate of change techniques to analyze the oximeter signal, to limit the effect of noise and motion artifact.

US-A-5 355 882 discloses the generic term of claim 1 and feature (a). The known method also comprises predicting absorbance and deriving a corrected absorbance based on a measured and predicted absorbance.

The present invention provides a technique for reducing noise effects in a system for measuring a physiological parameter, for example blood oxygen saturation, in which varying weights are assigned to different measurements, and the weighted measurements are averaged to obtain a filtered measurement.

Accordingly, in one aspect, the invention provides a method of reducing noise effects in a system for measuring a physiological parameter, the method comprising the steps of:

(a) generating a plurality of measurements derived from at least one wavelength of electromagnetic energy transmitted through living tissue;

(b) comparing selected measurements with at least one expected measurement characteristic;

(c) assigning one of a plurality of variable weights to each selected measurement based on the comparing step thereby generating a plurality of differently weighted measurements; and

(d) averaging a plurality of the differently weighted measurements to obtain a filtered measurement for use in estimating the physiological parameter.

In another aspect, the invention provides apparatus for reducing noise effects in a system for measuring a physiological parameter, comprising:

(a) means for generating a plurality of measurements derived from at least one wavelength of electromagnetic energy transmitted through living tissue;

(b) means for comparing selected measurements with at least one expected measurement characteristic;

(c) means for assigning one of a plurality of variable weights to each selected measurement based on the comparing step thereby generating a plurality of differently weighted measurements; and

(d) means for averaging a plurality of the differently weighted measurements to obtain a filtered measurement for use in estimating the physiological parameter.

Preferably, the apparatus includes means for calibrating the system to measure the physiological parameter in response to a signal indicative of the at least one wavelength of electromagnetic energy, for example as disclosed in US-4621643, US-4700708 and US-4770179

Preferably, the apparatus includes a sensor with an emitter for the said at least one wavelength of electromagnetic energy, sensing means for sensing the electromagnetic energy and for generating a first signal representative thereof, means for detachably coupling the sensor to an oximeter and for communicating signals between the sensor and the oximeter, and means for providing a second signal indicative of the at least one wavelength of electromagnetic energy.

The present invention makes use of filtering techniques which use mathematical models to describe how physiological parameters change in time, and how these parameters relate to measurement in a noisy environment. Such filters can modify a set of averaging weights and averaging times to arrive at an optimised estimation of the physiological parameter.

The technique of the invention can be used in conjunction with a pulse oximeter to determine the oxygen saturation of haemoglobin in arterial blood. A band-pass filter can be used to attenuate data below 0.5 Hz and above 10 Hz in order to remove out of band noise, at least partially. Filtered data can then be processed using a saturation calculation algorithm as discussed in more detail below.

The invention can employ adaptive filtering techniques (for example Kalman filtering) to calculate blood oxygen saturation. Kalman filtering allows one to fit parameters in a least squares sense when the parameters are varying in time. Traditionally one might employ a classical least squares (CLS) approach with low-pass filtering or averaging of the estimated quantity. Kalman filtering achieves substantially the same result, but the Kalman filter calculates the optimal amount of averaging. Appropriate Kalman filter algorithms are disclosed in *Introduction to Random Signals and Applied Kalman Filtering,* Second edition (1992), by R G Brown and P Y C Hwang, published by John Wiley & Sons. A Kalman cardiac gated averaging processor employing Kalman filter theory can be used in the calculation of oxygen saturation, in which the processor can be gated by triggers based on the pulse rate, for example supplied by an algorithm for calculating the pulse rate, or from an ECG wave form.

Using a Kalman filter, the method of the invention involves transforming appropriately pre-processed data into quantities corresponding to the oxyhaemglobin and total haemoglobin concentrations using appropriate extinction coefficients. The instantaneous ratio of these two transformed quantities gives the saturation. The instantaneous saturation value may be calculated directly by using the extinction coefficients, or from the ratio of ratios. The method need not search for maxima or minima like a pulse searching algorithm (although maxima or minima could be used and Kalman filtered if desired). Using instantaneous ratios (that is, a time based algorithm) rather than maxima/minima ratios (that is, an event based algorithm) keeps the code from being event driven and having to qualify data as it arrives. The method of the present invention therefore has the advantage of being simpler to implement than a pulse-searching event-based saturation calculation algorithm.

Preferably, the number of the differently weighted measurements which are averaged varies in response to the assigning step.

Preferably, a first number of differently weighted measurements are averaged to obtain the filtered measurement, the first number varying according to weights assigned to a plurality of successive differently weighted measurements in the assigning step. It is preferred then that a plurality of filtered measurements are generated for each wavelength, over time.

Preferably, the plurality of variable weights comprises first and second sets of measurements corresponding to first and second wavelengths, respectively. The steps of comparing and assigning are then performed on the first set of measurements. It is preferred that the method includes the step of assigning to the second set of measurements weights identical to those assigned to the first set of measurements, thereby obtaining separate filtered measurements for each of the first and second wavelengths.

Preferably, the plurality of measurements are obtained by combining data from at least two wavelengths.

Preferably, the variable weight assigned to each selected measurement is based in part on a rate of change between the selected measurement and a previous measurement.

Preferably, the generating step of the method comprises:

(a) taking the logarithm of a signal representative of the at least one wavelength of electromagnetic energy, thereby generating a first signal; and

(b) band pass filtering the first signal, thereby generating a second signal from which the plurality of measurements are derived.

Preferably, the generating step then includes the step of normalizing the second signal to generate a third signal from which the plurality of measurements are derived. The generating step can then include the step of taking the derivative of the third signal to generate a fourth signal from which the plurality of measurements are derived.

Preferably, the similarity between each selected measurement and the corresponding previous measurement is compared to an expected value. The expected value might correspond to a physiological model. It might correspond to a rate of change between a plurality of corresponding previous measurements.

Preferably, the corresponding previous measurement, with which the selected measurement is compared for assigning the variable weights, corresponds to a filtered measurement.

Preferably, the at least one expected measurement characteristic comprises a prediction corresponding to at least one previous filtered measurement.

Preferably, the selected measurements comprise digital signals.

Preferably, the plurality of measurements generated in step (a) of the method correspond to a series of cardiac pulses.

Preferably, the variable weights of the generated weighted measurements comprise a plurality of different non-zero numbers.

Preferably, the plurality of measurements generated in step (a) of the method are time-based and not event driven. Each filtered time-based measurement might then correspond to at least one of the following:

1. A current value of a ratio of ratios which is representative of oxygen saturation of haemoglobin in arterial blood.

2. A current value of oxygen saturation of haemoglobin in arterial blood. The at least one expected measurement characteristic can then comprise a constant representative of a rate of change of the oxygen saturation value.

The plurality of time-based measurements can be normalized prior to the comparing step, thereby reducing noise effects corresponding to motion artifact on some of the time-based measurements.

Preferably, the method of the invention includes the step of providing a signal indicative of the at least one wavelength of electromagnetic energy

Preferably, the method of the invention includes the step of filtering data corresponding to the wavelength of electromagnetic energy so that motion and noise energy not at integer multiples of a heart rate of the patient are attenuated.

The invention can involve reduction of noise effects when measuring a physiological parameter. It can include apparatus for reducing the noise effects which comprises:

means for generating a plurality of measurements derived from at least one wavelength of electromagnetic energy transmitted through living tissue;
means for providing a signal indicative of the at least one wavelength of electromagnetic energy;
means for comparing selected measurements with at least one expected measurement characteristic;
means for assigning one of a plurality of variable weights to each selected measurement based on the comparing step thereby generating a plurality of differently weighted measurements for each wavelength, the variable weights being assigned, in part, in response to a similarity between each selected measurement and a corresponding previous measurement, the variable weights comprising a plurality of different non-zero numbers;
means for averaging a plurality of the differently weighted measurements to obtain a filtered measurement for use in estimating the physiological parameter; and
means for calibrating the system to measure the physiological parameter in response to the signal indicative of the at least one wavelength of electromagnetic energy.

The invention also includes a monitor for measuring a physiological parameter, the monitor being for use with a sensor having emitting means for emitting at least one wavelength of electromagnetic energy, sensing means for sensing the electromagnetic energy and for generating a first signal representative thereof, means for detachably coupling the sensor to the oximeter and for providing communication of signals between the sensor and the oximeter, and means for providing a second signal indicative of the at least one wavelength of electromagnetic energy, the monitor comprising:

means for generating a plurality of measurements derived from the first signal;
means for comparing selected measurements with at least one expected measurement characteristic;
means for assigning one of a plurality of variable weights to each selected measurement based on the comparing step thereby generating a plurality of differently weighted measurements, the variable weights being assigned, in part, in response to a similarity between each selected measurement and a corresponding previous measurement, the variable weights comprising a plurality of different non-zero numbers;
means for averaging a plurality of the differently weighted measurements to obtain a filtered measurement for use in estimating the physiological parameter; and
means for calibrating the monitor to measure the physiological parameter in response to the second signal.

The present invention will now be described, by way of example only, with reference to the accompanying drawings, in which:

Figure 1 is a schematic representation of apparatus for measuring a physiological parameter such as oxygen saturation of haemoglobin of a patient;

Figure 2 is a block diagram illustrating the flow of data in apparatus such as that shown in Figure 1;

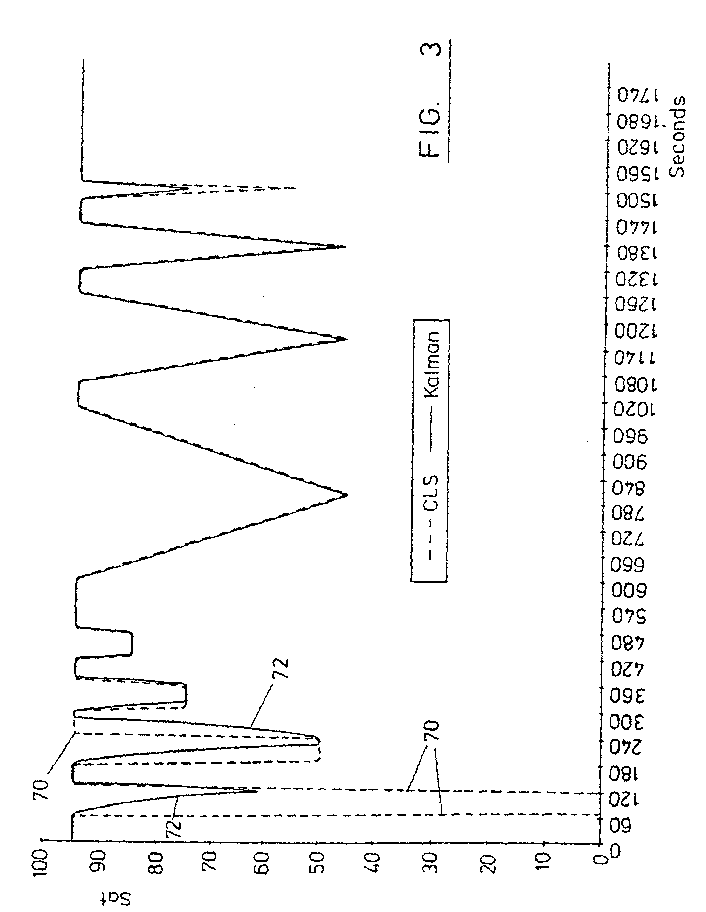

Figure 3 is a graph comparing the performance of a classic least squares algorithm to that of the Kalman algorithm;

Figure 4 is a graph comparing the inputs and outputs of the Kalman cardiac gated averaging filter.

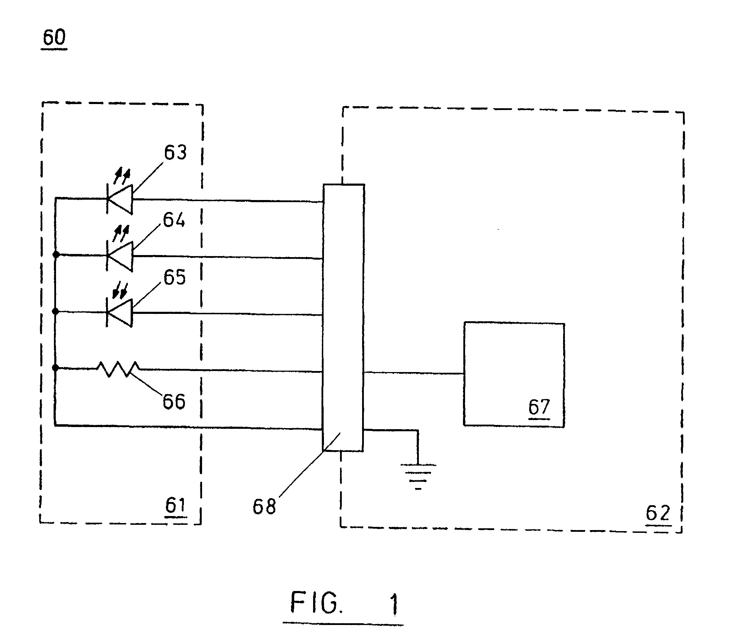

Referring to the drawings, Figure 1 shows apparatus for measuring physiological parameters such as oxygen saturation of haemoglobin of a patient. A sensor/oximeter combination 60 comprises a sensor 61 and an oximeter monitor 62. Sensor 61 includes LEDs 63 and 64 typically having wavelength emission characteristics in the infrared and red ranges of the spectrum, respectively. Photodiode sensor 65 receives the light transmitted by LEDs 63 and 64. Resistor 66 (or a similar electrical impedance reference) is chosen to correspond to a specific wavelength or combination of wavelengths as specified by a table relating impedance values to wavelengths. Decoding means 67 determines the impedance value of resistor 66, and appropriate extinction coefficients are generated which correspond to the transmission characteristics of the particular sensor 61. Thus, the oximeter may be used with a variety of sensors having LEDs which emit varying wavelengths of light without recalibration. The sensor 61 is detachably coupled to oximeter monitor 62 by means of a connector. An example of such a sensor/oximeter combination is disclosed in US-4621643.

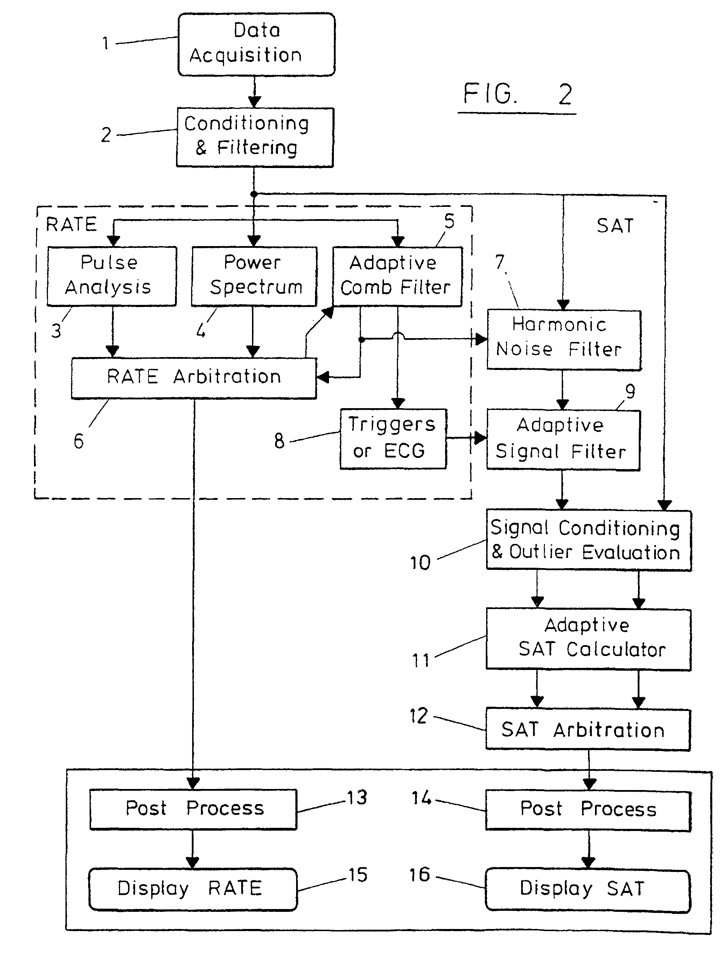

The data received from the sensor is processed according to the scheme shown in Figure 2. It can be processed using apparatus of the type disclosed in US-5348004. In initial process steps 1, 2, the natural logarithm of the data (usually from red and infra red LEDs) is taken, and the data is band pass filtered (step 1). The filtered data can then processed by algorithms for calculation of oxygen saturation. The algorithms for processing the filtered data can make use of Kalman filtering (step 11), with and without cardiac gated averaging (step 9). These filtering techniques are discussed in the "Introduction to Random Signals and Applied Kalman Filtering" publication mentioned above.

Using Kalman filtering, a parameter $x$ to be estimated (for example, oxygen saturation or pulse rate) varies in time in some predictable way. If the value of $x$ is known at some sample in time, then in the next sample, $x$ may be expected to have little or no variation from the previous value. $Q$ is the variance of this difference. The parameter $x$ is not measured directly. What is actually measured is a parameter $z$ which equals $x$ times a constant $H$ plus measurement noise. $R$ is the variance of this measurement noise. Rewriting these

$$x_k = x_{k-1} + n_k^Q$$

$$z_k = H_k x_k + n_k^R$$

The ability to estimate the value of $x$ knowing $z$ and the last estimate of $x$ is related to the two noises quantified by $R$ and $Q$. The Kalman filter quantifies the two noises in a parameter called the estimation error, $P$. The Kalman filter also uses an intermediate term called the Kalman gain, $K$. $P_0^{-1}$ is initialized with a value of zero. Then at each new data point $k$, the following steps are performed:

$$P_k^{-1} = P_{k-1}^{-1} + H_k^2 R_k^{-1}$$

$$K_k = P_k H_k R_k^{-1}$$

$$x_k = x_{k-1} + K_k(z_k - H_k x_{k-1})$$

$$P_{k+1} = P_k + Q_k$$

With the Kalman filter (step 11), the saturation is allowed to vary, and the model is separated into two parts. The first part is

$$v_k = u_k s_k + n_k^R$$

That is, the ratio of the transformed pre-processed data is the saturation value except for measurement noise. The spread of the data gives a real-time measurement of the noise variance. The second part says that on average saturation does not change in time, but if it does change the standard deviation of the change is some constant, $Q^{1/2}$ observable rate of change. That is, the second equation is

$$s_k = s_{k-1} + n_k^Q$$

This second equation gives the Kalman filter the ability to recognize that if saturation changes by 10 points in two seconds, for example, it must be due to measurement noise. The Kalman filter then averages the calculated saturation more with previous values to bring the change more in line with what is expected from physiology. In contrast, if the change is within bounds the Kalman filter will average very little.

The value of *R* is estimated from the difference between *v* and us over the last N points, where the user specifies the value N. In one embodiment, the Kalman model for saturation also gives less weight to the smaller portions of a pulse, more weight to the larger portions, and adds a small incremental value to the actual variance to represent the error inherent in the measurement system (for example, hardware noise).

In a second Kalman filter (step 12), the Kalman filter limits the changes to the time derivative of saturation. The equations for this filter say that the expected value of the time derivative of saturation should statistically be unchanged with time.

$$\frac{dx_k}{dt} = \frac{dx_{k-1}}{dt} + n_k^Q$$

$$\frac{dz_k}{dt} = \frac{dx_k}{dt} + n_k^R$$

where *z* is the estimate of saturation from the first Kalman filter, and *x* is the estimate of saturation after limiting the changes to its time derivative. In this embodiment, the parameter $n^Q$ is preferred to be chosen to correspond to the second derivative of the observed rate of change, and $n^R$ is estimated from the data. In the general form of the Kalman filter, these two separate filters could be combined into one filter. By separating them, the need to use matrix algebra is eliminated and each Kalman filter is able to be tested separately.

The measurement noise is estimated by centring a window around the data values being used. This centring gives a more accurate estimate of the noise, but delays the output of the Kalman filter by half the window length. When one second window is used, it appears that the filter can respond quickly to motion coming and going, and the one-half second delay in saturation estimation is not clinically significant.

Kalman filtering with cardiac gated averaging

A Kalman CGA algorithm can be applied in series with a Kalman saturation algorithm (steps 8, 9). The Kalman CGA processor optimally averages successive plethysmograph pulses or waveforms to create an optimally filtered plethysmograph waveform. The first equation below correlates the measured plethysmograph shape with the averaged plethysmograph wave shape except for measurement noise.

$$z_k = x_k + n_k^Q$$

The value of *Q* is estimated from the data. According to the following equation, the new pulse cannot differ from the averaged plethysmograph pulse by more than some acceptable percentage.

$$x_k = x_{k-N} + n_k^R$$

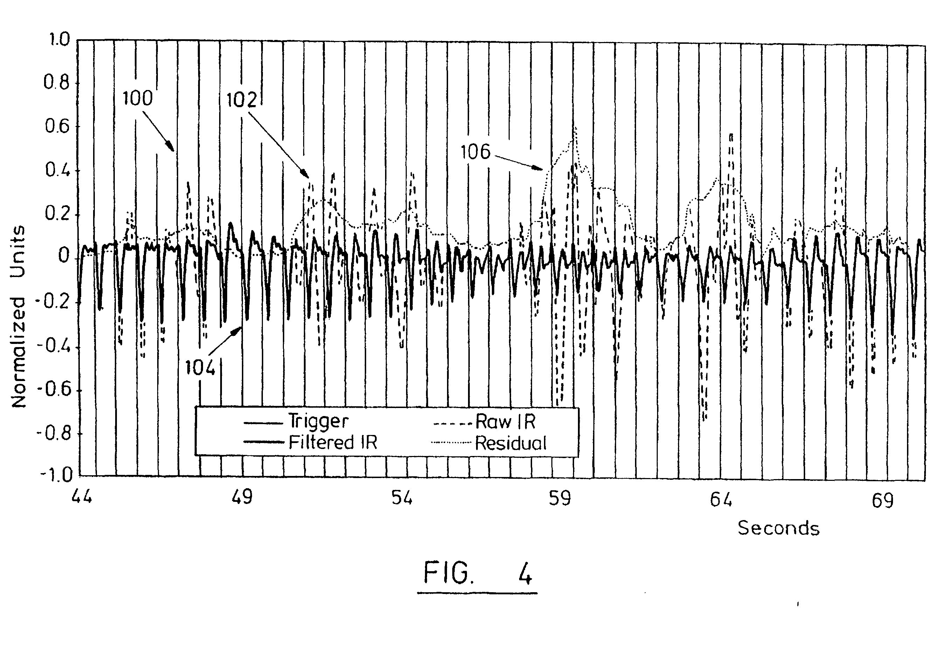

The Kalman cardiac gated averaging model automatically averages more data points if the incoming wave form varies significantly, yet has the ability to update quickly if the wave form obeys assumptions based on expected physiological variation. The Kalman cardiac gated averaging represents a significant improvement over prior art methods of calculating saturation as used in oximeters available from Nellcor Incorporated under the trade marks N200 and N3000, and as disclosed in US-4802486. Figure 4 shows an example of the inputs and outputs of a Kalman filter according to one embodiment of the invention. The trigger waveform 100 is from the R-wave of an ECG or from a pulse rate calculation method. The raw data waveform 102 is at times quite corrupted by motion, yet by variable averaging, the Kalman cardiac gated averaging technique is able to keep the filtered waveform 104 looking quite regular. The estimated residual 106 correlates well in time with the noise on the measured data.

It will be understood that the Kalman cardiac gated averaging technique may be used to shape the oximetry data pulses for processing by either a CLS saturation calculation technique, the Kalman saturation calculation technique, or an alternate technique. Either embodiment could use an ECG pulse rate, or a pulse rate generated by an algorithm which processes oximeter data as the cardiac gated averaging trigger. The Kalman saturation calculation technique may be used without the Kalman cardiac gated averaging technique.

Referring again to Figure 2, two saturation values are calculated from the data from the band pass filter. One saturation value is obtained using a harmonic filter (step 7) and a Kalman filter with cardiac gated averaging, using triggers from an ECG waveform. The harmonic filter (step 7) digitally filters the IR and red waveforms such that only energy at integer multiples of the heart rate is allowed through the filter. The response of harmonic filter (step 7) varies with the heart rate signal supplied to it to attenuate motion and noise energy not at the heart rate. In this arrangement, the subsequent filtering by Kalman CGA (step 9) and/or the saturation calculation (step 11) algorithm described below applies the same weighting and averaging to both the IR and red data streams on the basis of the filtered data stream.

The second saturation value is obtained by application of a Kalman filter (step 11). In contrast to the first Kalman filter with cardiac gated averaging which is event based, the second filter is time based. The second filter operates on data from the band pass filter and on data from the first filter. Prior to application of the second filter, data points resulting in an impossible saturation calculation (for example negative saturation) are rejected (step 10). After application of the second filter, the best saturation value is chosen according to confidence levels associated with each value (step 12).

The saturation value after the second filter is displayed after appropriate post-processing to determine whether and how it is to be displayed (step 14). Confidence levels in the oxygen saturation can be estimated from metrics available from the algorithms performed on the oximeter data, determining which saturation can be considered reliable. For example, the confidence level can be determined dependent on the age of the signal from which the saturation level is calculated and the deviation of that level from an estimated value.

## Claims

1. A method of reducing noise effects in a system for measuring a physiological parameter, the method comprising the steps of:

   (a) generating a plurality of measurements derived from at least one wavelength of electromagnetic energy transmitted through living tissue;

   (b) comparing selected measurements with at least one expected measurement characteristic;

   (c) assigning one of a plurality of variable weights to each selected measurement based on the comparing step thereby generating a plurality of differently weighted measurements; and

   (d) averaging a plurality of the differently weighted measurements to obtain a filtered measurement for use in estimating the physiological parameter.

2. A method as claimed in claim 1, in which a number of the differently weighted measurements averaged varies in response to the assigning step.

3. A method as claimed in claim 1, in which a first number of differently weighted measurements are averaged to obtain the filtered measurement, the first number varying according to weights assigned to a plurality of successive differently weighted measurements in the assigning step, and wherein, over time, a plurality of filtered measurements are generated for each wavelength.

4. A method as claimed in claim 1, in which the plurality of measurements comprises first and second sets of measurements corresponding to first and second wavelengths, respectively, and wherein the steps of comparing and assigning are performed on the first set of measurements, and further comprising the step of assigning to the second set of measurements weights identical to those assigned to the first set of measurements, thereby obtaining separate filtered measurements for each of the first and second wavelengths.

5. A method as claimed in claim 1, in which the plurality of variable weights are obtained by combining data from at least two wavelengths.

6. A method as claimed in claim 1, in which the variable weight assigned to each selected measurement is based in part on a rate of change between the selected measurement and a previous measurement.

7. A method as claimed in claim 1, in which said generating step comprises:

   (a) taking the logarithm of a signal representative of the at least one wavelength of electromagnetic energy, thereby generating a first signal; and

   (b) band pass filtering the first signal, thereby generating a second signal from which the plurality of measurements are derived.

8. A method as claimed in claim 7, in which said generating step further comprises normalizing the second signal, thereby generating a third signal from which the plurality of measurements are derived.

9. A method as claimed in claim 8, in which said generating step further comprises taking the derivative of the third signal, thereby generating a fourth signal from which the plurality of measurements are derived.

10. A method as claimed in claim 1, in which the similarity between each selected measurement and the corresponding previous measurement is compared to an expected value.

11. A method as claimed in claim 10, in which the expected value corresponds to a physiological model.

12. A method as claimed in claim 10, in which the expected value corresponds to a rate of change between a plurality of corresponding previous measurements.

13. A method as claimed in claim 1, in which the corresponding previous measurement corresponds to a filtered measurement.

**14.** A method as claimed in claim 1, in which the at least one expected measurement characteristic comprises a prediction corresponding to at least one previous filtered measurement.

**15.** A method as claimed in claim 1, in which the selected measurements comprise digital signals.

**16.** A method as claimed in claim 1, in which the plurality of measurements generated in step (a) correspond to a series of cardiac pulses.

**17.** A method as claimed in claim 1, in which the variable weights of the said generated weighted measurements comprise a plurality of different non-zero numbers.

**18.** A method as claimed in claim 1, in which the plurality of measurements generated in step (a) are time-based and not event driven.

**19.** A method as claimed in claim 18, in which each filtered time-based measurement corresponds to a current value of a ratio of ratios which is representative of oxygen saturation of haemoglobin in arterial blood.

**20.** A method as claimed in claim 18, in which each filtered time-based measurement corresponds to a current value of oxygen saturation of haemoglobin in arterial blood.

**21.** A method as claimed in claim 20, in which the at least one expected measurement characteristic comprises a constant representative of a rate of change of the oxygen saturation value.

**22.** A method as claimed in claim 18, in which the plurality of time-based measurements are normalized prior to the comparing step, thereby reducing noise effects corresponding to motion artifact on some of the time-based measurements.

**23.** A method as claimed in claim 1, which includes providing a signal indicative of the at least one wavelength of electromagnetic energy

**24.** A method as claimed in claim 1, which includes the step of filtering data corresponding to the said wavelength of electromagnetic energy so that motion and noise energy not at integer multiples of a heart rate of the patient are attenuated.

**25.** Apparatus for reducing noise effects in a system for measuring a physiological parameter, comprising:

(a) means for generating a plurality of measurements derived from at least one wavelength of electromagnetic energy transmitted through living tissue;

(b) means for comparing selected measurements with at least one expected measurement characteristic;

(c) means for assigning one of a plurality of variable weights to each selected measurement based on the comparing step thereby generating a plurality of differently weighted measurements; and

(d) means for averaging a plurality of the differently weighted measurements to obtain a filtered measurement for use in estimating the physiological parameter.

**26.** Apparatus as claimed in claim 25, which includes means for calibrating the system to measure the physiological parameter in response to a signal indicative of the at least one wavelength of electromagnetic energy.

**27.** Apparatus as claimed in claim 25, which includes a sensor with an emitter for the said at least one wavelength of electromagnetic energy, sensing means for sensing the electromagnetic energy and for generating a first signal representative thereof, means for detachably coupling the sensor to an oximeter and for communicating signals between the sensor and the oximeter, and means for providing a second signal indicative of the at least one wavelength of electromagnetic energy.

**Patentansprüche**

**1.** Verfahren zur Verringerung von Rauscheffekten in einem System zur Messung eines physiologischen Parameters, wobei das Verfahren die Schritte umfasst:

(a) Erzeugen einer Mehrzahl von Messungen, die von mindestens einer Wellenlänge elektromagnetischer Energie abgeleitet sind, welche durch lebendes Gewebe übertragen wird;
(b) Vergleichen von ausgewählten Messungen mit mindestens einer erwarteten Messcharakteristik;
(c) Zuordnen eines einer Mehrzahl von variablen Gewichten zu jeder ausgewählten Messung auf der Grundlage des Vergleichsschrittes, wodurch eine Mehrzahl von verschieden gewichteten Messungen erzeugt wird; und
(d) Mitteln einer Mehrzahl der verschieden gewichteten Messungen, um eine gefilterte Messung zur Verwendung bei der Abschätzung des physiologischen Parameters zu erhalten.

**2.** Verfahren nach Anspruch 1, in dem eine Zahl der verschieden gewichteten gemittelten Messungen in Abhängigkeit von dem Zuordnungsschritt variiert.

**3.** Verfahren nach Anspruch 1, in dem eine erste Zahl von verschieden gewichteten Messungen gemittelt wird, um die gefilterte Messung zu erhalten, wobei die erste Zahl gemäß den Gewichten variiert, die einer Mehrzahl von aufeinander folgenden verschieden gewichteten Messungen in dem Zuordnungsschritt zugeordnet wurde, und wobei über die Zeit eine Mehrzahl von gefilterten Messungen für jede Wellenlänge erzeugt wird.

**4.** Verfahren nach Anspruch 1, in dem die Mehrzahl von Messungen einen ersten und einen zweiten Satz von Messungen umfasst, die einer ersten bzw. zweiten Wellenlänge entsprechen, und wobei die Schritte des Vergleichs und der Zuordnung bei dem ersten Satz von Messungen durchgeführt werden, und weiter umfassend den Schritt der Zuordnung von Gewichten zu dem zweiten Satz von Messungen, welche identisch sind mit denjenigen, die dem ersten Satz von Messungen zugeordnet wurden, wodurch getrennte gefilterte Messungen für sowohl die erste als auch die zweite Wellenlänge erhalten werden.

**5.** Verfahren nach Anspruch 1, in dem die Mehrzahl der variablen Gewichte erhalten wird, indem man die Daten aus mindestens zwei Wellenlängen kombiniert.

**6.** Verfahren nach Anspruch 1, in dem das variable Gewicht, das jeder ausgewählten Messung zugeordnet wird, teilweise auf einer Änderungsrate zwischen der ausgewählten Messung und einer vorangehenden Messung beruht.

**7.** Verfahren nach Anspruch 1, in dem der Erzeugungsschritt umfasst:

(a) Verwenden des Logarithmus eines Signals, das für die mindestens eine Wellenlänge elektromatischer Energie repräsentativ ist, wodurch ein erstes Signal erzeugt wird; und
(b) Bandpass-Filtern des ersten Signals, wodurch ein zweites Signal erzeugt wird, aus dem die Mehrzahl von Messungen abgeleitet wird.

**8.** Verfahren nach Anspruch 7, in dem der Erzeugungsschritt weiter die Normierung des zweiten Signals umfasst, wodurch ein drittes Signal erzeugt wird, aus dem die Mehrzahl von Messungen abgeleitet wird.

**9.** Verfahren nach Anspruch 8, in dem der Erzeugungsschritt weiter die Verwendung der Ableitung des dritten Signals umfasst, wodurch ein viertes Signal erzeugt wird, aus dem die Mehrzahl von Messungen abgeleitet wird.

**10.** Verfahren nach Anspruch 1, in dem die Ähnlichkeit zwischen jeder ausgewählten Messung und der entsprechenden vorangehenden Messung mit einem erwarteten Wert verglichen wird.

**11.** Verfahren nach Anspruch 10, in dem der erwartete Wert einem physiologischen Modell entspricht.

**12.** Verfahren nach Anspruch 10, in dem der erwartete Wert einer Änderungsrate zwischen einer Mehrzahl von entsprechenden vorangehenden Messungen entspricht.

**13.** Verfahren nach Anspruch 1, in dem die entsprechende vorangehenden Messung einer gefilterten Messung entspricht.

**14.** Verfahren nach Anspruch 1, in dem die mindestens eine erwartete Messcharakteristik eine Vorhersage umfasst, welche mindestens einer vorangehenden gefilterten Messung entspricht.

**15.** Verfahren nach Anspruch 1, in dem die ausgewählte Messung digitale Signale umfasst.

**16.** Verfahren nach Anspruch 1, in dem die Mehrzahl von Messungen, die in Schritt (a) erzeugt werden, einer Reihe von Herzpulsen entspricht.

**17.** Verfahren nach Anspruch 1, in dem die variablen Gewichte der erzeugten gewichteten Messungen einer Mehrzahl von verschiedenen Zahlen ungleich Null entsprechen.

**18.** Verfahren nach Anspruch 1, in dem die Mehrzahl von Messungen, die in Schritt (a) erzeugt werden, auf einer Zeit-Basis beruhen und nicht durch ein Ereignis angetrieben sind.

**19.** Verfahren nach Anspruch 18, in dem jede gefilterte Messung auf Zeit-Basis einem aktuellen Wert eines Verhältnisses von Verhältnissen entspricht, welches für die Sauerstoffsättigung von Hämoglobin in arteriellem Blut repräsentativ ist.

**20.** Verfahren nach Anspruch 18, in dem jede gefilterte Messung auf Zeit-Basis einem aktuellen Wert der Sauerstoffsättigung von Hämoglobin in arteriellem Blut entspricht.

**21.** Verfahren nach Anspruch 20, in dem die mindestens eine erwartete Messcharakteristik einen konstanten Repräsentanten einer Änderungsrate des Sauerstoffsättigungswerts umfasst.

22. Verfahren nach Anspruch 18, in dem die Mehrzahl der Messungen auf Zeit-Basis vor dem Vergleichsschritt normiert wird, wodurch Rauscheffekte verringert werden, die einem Bewegungsartefakt bei einigen der Messungen auf Zeit-Basis entsprechen.

23. Verfahren nach Anspruch 1, welches die Bereitstellung eines Signals umfasst, das die mindestens eine Wellenlänge elektromagnetischer Strahlung anzeigt.

24. Verfahren nach Anspruch 1, welches den Schritt des Filterns von Daten umfasst, die der Wellenlänge elektromagnetischer Strahlung entsprechen, so dass Bewegungs- und Rauschenergie, die nicht ganze Vielfache einer Herzfrequenz des Patienten ist, gedämpft wird.

25. Vorrichtung zur Verringerung von Rauscheffekten in einem System zur Messung eines physiologischen Parameters, umfassend:

(a) Mittel zum Erzeugen einer Mehrzahl von Messungen, die von mindestens einer Wellenlänge elektromagnetischer Strahlung abgeleitet sind, welche durch lebendes Gewebe übertragen wird;

(b) Mittel zum Vergleichen ausgewählter Messungen mit mindestens einer erwarteter Messcharakteristik;

(c) Mittel zum Zuordnen von einem einer Mehrzahl von variablen Gewichten zu jeder ausgewählten Messung auf der Grundlage des vergleichenden Schrittes, wodurch eine Mehrzahl von verschieden gewichteten Messungen erzeugt wird; und

(d) Mittel zum Mitteln einer Mehrzahl der verschieden gewichteten Messungen, um eine gefilterte Messung zur Verwendung bei der Abschätzung des physiologischen Parameters zu erhalten.

26. Vorrichtung nach Anspruch 25, welche Mittel zum Kalibrieren des Systems umfasst, um den physiologischen Parameter in Abhängigkeit von einem Signal zu messen, das die mindestens eine Wellenlänge elektromagnetischer Energie anzeigt.

27. Vorrichtung nach Anspruch 25, welche einen Messfühler mit einer Sendeeinrichtung für die mindestens eine Wellenlänge elektromagnetischer Energie, Erfassungsmittel zum Erfassen der elektromagnetischen Energie und zum Erzeugen eines ersten Signals, welches dafür repräsentativ ist, Mittel zum lösbaren Koppeln des Messfühlers an ein Oximeter und zur Kommunikation von Signalen zwischen dem Messfühler und dem Oximeter und Mittel zur Bereitstellung eines zweiten Signals, das die mindestens eine Wellenlänge elektromagnetischer Energie anzeigt, umfasst.

## Revendications

1. Procédé pour réduire des effets de bruit dans un système pour mesurer un paramètre physiologique, le procédé comprenant les étapes consistant à:

(a) produire une pluralité de mesures obtenues à partir d'au moins une longueur d'onde d'une énergie électromagnétique transmise dans un tissu vivant;
(b) comparer des mesures sélectionnées à au moins une caractéristique de mesure attendue;
(c) affecter l'une d'une pluralité de poids variables à chaque mesure sélectionnée sur la base de l'étape de comparaison en produisant ainsi une pluralité de mesures pondérées différemment; et
(d) former la moyenne d'une pluralité des mesures pondérées différemment pour l'obtention d'une mesure filtrée destinée à être utilisée pour estimer le paramètre physiologique.

2. Procédé selon la revendication 1, dans lequel, un certain nombre des mesures pondérées différemment, dont la moyenne est formée, varient en référence avec l'étape d'affectation.

3. Procédé selon la revendication 1, selon lequel la moyenne d'un premier nombre de mesures pondérées différemment est formée pour l'obtention de la mesure filtrée, le premier nombre variant en fonction de poids affectés à une pluralité de mesures successives pondérées différemment lors de l'étape d'affectation, et selon lequel; dans le temps, une pluralité de mesures filtrées sont produites pour chaque longueur d'onde.

4. Procédé selon la revendication 1, selon lequel la pluralité de mesure comprend des premier et second ensembles de mesures correspondant respectivement à des première et seconde longueurs d'onde, et selon lequel les étapes de comparaison et d'affectation sont appliquées au premier ensemble de mesures, et comprenant en outré l'étape consistant à affecter au second ensemble de mesures, des poids identiques à ceux affectés au premier ensemble de mesures, ce qui permet d'obtenir des mesures filtrées séparées pour chacune des première et seconde longueurs d'onde.

5. Procédé selon la revendication 1, selon lequel la pluralité de poids variables sont obtenus en combinant des données provenant d'au moins deux longueurs d'onde.

6. Procédé selon la revendication 1, selon lequel le poids variable affecté à chaque mesure sélectionnée est basé en partie sur une vitesse de variation entre la mesure sélectionnée et une mesure précédente.

7. Procédé selon la revendication 1, selon lequel ladite étape de production comprend:

(a) la prise du logarithme d'un signal représentatif de la au moins une longueur d'onde d'énergie électromagnétique, qui permet de produire un premier signal; et
(b) un filtrage passe-bande du premier signal ce qui permet de produire un second signal, d'où sont dérivées la pluralité de mesures.

8. Procédé selon la revendication 7, selon lequel ladite étape de production comporte en outre la normalisation du second signal, ce qui permet de produire un troisième signal d'où sont dérivées la pluralité de mesures.

9. Procédé selon la revendication 8, selon lequel ladite étape de production comprend en outre la formation de la dérivée du troisième signal, ce qui permet de produire un quatrième signal d'où sont -dérivées la pluralité de mesures.

10. Procédé selon la revendication 1, selon lequel la similitude entre chaque mesure sélectionnée et la mesure précédente correspondante est la mesure précédente correspondante est comparée à une valeur attendue.

11. Procédé selon la revendication 10, selon lequel la valeur attendue correspond à un modèle physiologique.

12. Procédé selon la revendication 10, selon lequel la valeur attendue correspond à une vitesse de variation entre une pluralité de mesures précédentes correspondantes.

13. Procédé selon la revendication 1, selon lequel la mesure précédente correspondante correspond à une mesure filtrée.

14. Procédé selon la revendication 1, selon lequel la au moins une caractéristique de mesure attendue comprend une prédiction correspondant au moins à une mesure filtrée précédente.

15. Procédé selon la revendication 1, selon lequel les mesures sélectionnées comprennent des signaux numériques.

16. Procédé selon la revendication 1, selon lequel la pluralité de mesures produites lors de l'étape (a) correspondent à une série d'impulsions cardiaques.

17. Procédé selon la revendication 1, selon lequel les poids variables desdites mesures pondérées produites comprennent une pluralité de nombre non, nuls différents.

18. Procédé selon la revendication 1, selon lequel la pluralité de mesures produites lors de l'étape (a) sont basées sur le temps et ne sont pas pilotées par l'évènement.

19. Procédé selon la revendication 18, selon lequel chaque mesure filtrée basée sur le temps correspond à une valeur actuelle d'un rapport de rapports, qui est représentatif de la saturation en oxygène de l'hémoglobine dans le sang artériel.

20. Procédé selon la revendication 18, selon lequel chaque mesure filtrée basée sur le temps correspond à une valeur actuelle de la saturation en oxygène de l'hémoglobine dans le sang artériel.

21. Procédé selon la revendication 20, selon lequel la au moins une caractéristique de mesure attendue comprend une constante représentative d'une vitesse de variation de la valeur de saturation en oxygène.

22. Procédé selon la revendication 18, selon lequel la pluralité de mesures basées sur le temps sont normalisées avant l'étape de comparaison, ce qui permet de réduire des effets de bruit correspondant à un artefact de déplacement sur certaines des mesures basées sur le temps.

23. Procédé selon la revendication 1, qui inclut le fait de prévoir un signal indicatif de la au moins une longueur d'onde de l'énergie électromagnétique.

24. Procédé selon la revendication 1, qui inclut l'étape consistant à filtrer des données correspondant à ladite longueur d'onde de l'énergie électromagnétique de telle sorte qu'une énergie de déplacement et une énergie de bruit, qui ne sont pas. des multiples entiers d'un rythme cardiaque du patient, sont atténuées.

25. Dispositif pour réduire des effets de bruit dans un système pour mesurer un paramètre physiologique, comprenant:

(a) des moyens pour produire une pluralité de mesures dérivées au moins d'une longueur d'onde d'une énergie électromagnétique transmise par le tissu vivant;

(b) des moyens pour comparer des mesures sélectionnées à au moins une caractéristique de mesure attendue;

(c) des moyens pour affecter l'un d'une pluralité de poids variables à chaque mesure sélectionnée sur la base de l'étape de comparaison, ce qui permet d'obtenir une pluralité de mesures pondérées différemment; et

(d) des moyens pour former la moyenne d'une pluralité des mesures pondérées différemment pour l'obtention d'une mesure filtrée pour son utilisation dans l'estimation du paramètre physiologique.

26. Dispositif selon la revendication 25, qui inclut des moyens pour étalonner le système afin de mesurer le paramètre physiologique en réponse à un signal indicatif de la au moins une longueur d'onde d'énergie électromagnétique.

27. Dispositif selon la revendication 25, qui inclut un capteur comportant un émetteur pour ladite au moins une longueur d'onde d'énergie électromagnétique, des moyens de détection pour détecter l'énergie électromagnétique, et pour produire un premier signal représentatif de cette énergie, des moyens pour coupler de façon amovible le capteur à un oxymètre et pour transmettre des signaux entre le capteur et l'oxymètre, et des moyens pour produire un second signal indicatif de la au moins une longueur d'onde d'énergie électromagnétique.

EP 1 006 863 B1

FIG. 1

FIG. 2

FIG. 3

EP 1 006 863 B1

FIG. 4